# NEW EUROPEAN PATENT SPECIFICATION

(11) **EP 0 512 090 B2**
(45) Date of publication and mention of the opposition decision: **22.11.2006**
(45) Mention of the grant of the patent: 02.01.1997
(21) Application number: 91920172.3
(22) Date of filing: 26.09.1991
(51) Int. Cl.: A61K 38/55, A61K 31/56

(54) **TREATMENT OF INFLAMMATION**
BEHANDLUNG VON ENTZÜNDUNGEN
TRAITEMENT D'INFLAMMATIONS

(30) Priority: 16.10.1990 US 598241; 18.01.1991 US 643727; 11.04.1991 US 683620
(43) Date of publication of application: 11.11.1992
(73) Proprietor: Sonoran Desert Chemicals LLC, Scottsdale, Arizona 85251 (US)
(72) Inventor: Lezdey, John, Cherry Hill, New Jersey 08034 (US); Wachter, Allan M., Tempe, Arizona 85284 (US)
(74) Representative: Bassett, Richard Simon
(86) International application number: PCT/US1991/006847
(87) International publication number: WO 1992/006706

(56) References cited:
- EP-A- 0 139 383
- EP-A- 0 289 336
- EP-A- 0 304 971
- EP-A- 0 432 117
- US-A- 4 916 117
- US-A- 5 008 242
- US-A- 5 093 316
- BIOLOGICAL CHEMISTRY HOPPE-SEYLER, vol. 371, Suppl., May 1990, Berlin (DE); S. C. DIETZE et al., pp. 75-79
- JOURNAL OF BIOLOGICAL CHEMISTRY (MICROFILMS), vol. 264, no. 35, 15 December 1989, Baltimore, MD, US, pp. 21308-21315; N.M. SCHECHTER et al.: "Reaction of Human Skin Chymotrypsin-like Proteinase Chymase with Plasma Proteinase Inhibitors"
- BIOCHEMICAL MEDICINE & METABOLIC BIOLOGY, vol. 38, 1987; FUKUSEN et al., pp. 165-169
- JOURNAL OF CLINICAL INVESTIGATION, vol. 71, June 1983; SCHLEIMER et al., pp. 1830-1835
- AMERICAN REVIEW OF RESPIRATORY DISEASES, vol. 135, suppl., 1987; WASSERMAN, pp. S46-S48
- CLINICAL IMMUNOLOGY & IMMUNOPATHOLOGY, vol. 50, suppl., 1989; MARONE et al., pp. S24-S40
- ANNALS OF ALLERGY, vol. 63, no. 6, Suppl., 1989; WASSERMAN, pp. S46-S50
- ANNUAL REVIEW OF IMMUNOLOGY, vol. 1, 1983; LARSEN et al., pp. 335-359
- Annals of Internal Medicine 1989, 111(3), pp. 206-212
- McElvaney et al., Clinical Research, vol. 38, no. 2, 485A (1990)

## Description

### Field of the Invention

The present invention relates to the treatment of mammals afflicted with mast cell implicated disease. More particularly, the present invention relates to the use of serine protease inhibitors, their analogs, salts or derivatives for the preparation of pharmaceutical compositions for the direct or prophylaxis treatment of certain mast cell implicated diseases, particularly inflammatory conditions in parents. There is particularly provided topical compositions for treating the symptoms of inflammatory skin conditions and compositions for treating pulmonary inflammation by inhalation therapy.

### Background of the Invention.

Prior to the present invention it was generally believed that serine protease inhibitors could be used only to supplement a deficiency occurring as a result of a genetic defect or a chemically produced deficiency resulting from an event such as smoking. Moreover, no consideration was previously given for directly controlling diseases in which mast cells are implicated by administering serine protease inhibitors when serum levels of proteases or protease inhibitors are normal. Mast cells have been found to be implicated in diseases and events such as allergic and non-allergic rhinitis, nasal polyposis, atopic dermatitis, including psoriasis, contact dermatitis, pancreatitis, emphysema, asthma, colitis, Crohn's Disease, wound healing, cluster headaches, coronary artery spasm, rheumatoid arthritis etc.

Inflammation is a non-specific response of tissues to diverse stimuli or insults and results in release of a variety of materials at the site of inflammation that induce pain. It is now recognized that mast cells are indicated in the patho-physiology of inflammatory skin conditions as well as in other physiological disorders. Mast cells provide the greatest source of histamines in acute inflammation. Mast cells have also been noted in hypertropic scars.

It is now recognized that in a certain injury or a disease neutrophils, mast cells, T-cells and their mediators induce an inflammatory state resulting in a localized imbalance of elevated serine proteases with a concominant deficiency of their naturally occurring inhibitors despite normal serine protease inhibitor serum levels. Mast cells are critical in recruiting the cells (eosinophils, basophils and neutrophils) involved in the late phase reaction (LPR). Mast cell and neutrophile mediators appear to have a central role in the LPR. Monocytes through the release of cytokines, interleukin - 1,6 and tumor necrosis factor further amplify the LPR. Platelet activating factor, a mediator from mast cells, neutrophils and platelets is a potent bronchoconstrictor. Histamines are also released by the degranulation of mast cells as well as leukotriene T4 (LTB4) which play an important role in asthma. IgE upon activation by an antagonist causes degranulation of mast cells. Alpha 1-antitrypsin inhibits the mediators of mast cells and neutrophils, and also regulates IgE biosynthesis. The T-cell lymphokine glycosylation enhancing factor (GEF) is a serine protease that has been shown to enhance IgE response. The serine protease inhibitors decrease mast cell mediator release by inhibiting local IgE biosynthesis and T-cell lymphokine production. Serine proteases not only activate kinins and complements but also mediate tissue necrosis. The serine proteases, elastase and cathepsin G, have been shown to stimulate the production of platelet activating factor and LTB4.

Eosinophils and neutrophils are prominent in inflammatory lesions due to the potent chemoattractants released by mast cells.

Neutrophils are a main source of serine elastase and cathepsin G which are important in the tissue damage of inflammation, especially in rheumatoid arthritis.

The most direct approach to therapy of inflammatory skin conditions appears to be a direct attack at the site of inflammation of the mediators of inflammation and pain and the reduction of those neutrophilic derivatives which can cause damage to the growth of new tissue during the healing process.

Alpha 2-macroglobulin is a glycoprotein containing 8-11% carbohydrate which can be isolated from plasma by gel filtration chromatography.

Alpha 1-proteinase inhibitor (alpha 1-antitrypsin) is a glycoprotein having a molecular weight of 53,000 determined by sedimentation equilibrium centrifugation. The glycoprotein consists of a single polypeptide chain to which several oligosaccharide units are covalently bonded. Human alpha 1-proteinase inhibitor has a role in controlling tissue destruction by endogenous serine proteinases. A genetic deficiency of alpha-1-proteinase inhibitor, which accounts for 90% of the trypsin inhibitory capacity in blood plasma, has been shown to be associated with the premature development of pulmonary emphysema. The degradation of elastin associated with emphysema probably results from a local imbalance of elastolytic enzymes and the naturally occurring tissue and plasma proteinase inhibitors. Alpha-1-proteinase inhibitor inhibits human pancreatic and leukocyte elastases. See Pannell et al, Biochemistry. 13,5339 (1974); Johnson et al, Biochem. Biophys. Res. Commun., 72 33 (1976); Del Mar et al, Biochem. Biophys. Res. Commun., 88, 346 (1979); and Heimburger et al, Proc. Int. Res. Conf. Proteinase Inhibitors. 1st. 1-21 (1970).

The article of Groutas entitled "Inhibitors of Leukocyte Elastase and Leukocyte Cathepsin G Agents for the Treatment of Emphysema and Related Ailments' medical Research Reviews, Vol. 7, No. 7, 227-241 (1987), discloses the role of eglin, elastinal 1 and elasnin in emphysema.

U.S. Pat. No. 4,732,973 to Barr et al discloses typical analogs of serine protease inhibitors which may be used in the present invention.

U.S. Patent No. 4,916,117 to Lezdey et al discloses the treatment of pulmonary inflammation with microcrystalline alpha-1-antichymatrypsin.

S.C. Dietze et al. in Biol. Chem. Hoppe-Seyler, vol. 371, Suppl. pp 75-79 (May 1990), see especially page 78, suggest that inhibitors of serine proteases with trypsin-like specificity have no effect on degranulation of and histamine release from mast cells. The prejudice expressed by this reference is overcome by the present invention.

EP-A1-0 432 117 by the present inventors refers to the prophylaxis or direct treatment of inflammation by the administering of alpha 1-antichymotrypsin, its salts or derivatives. In the compositions administered, alpha 1-antitrypsin may also be present, but never without the simultaneous presence of the alpha 1-antichymotrypsin.

It is understood that the term "serine protease inhibitors" as used herein refers to the inhibitors derived from a particular species and that inhibit the proteases of the same species. However, human serine protease inhibitors may be used in veterinary products but not vice versa.

### Summary of the invention

The present invention relates to the use of alpha 1-antitrypsin, secretory leucocyte protease inhibitor, C-reactive protein, serum amyloid A protein and/or alpha-2-macroglobulin, their analogs, salts or derivatives which inhibit the degranulation of mast cells and/or have an affinity to the mediators of mast cells, for the preparation of a pharmaceutical composition for administration to the site of disease or injury of diseases implicated by mast cells and their mediators, wherein the disease is asthma or a skin disease and, if the disease is a skin disease, then only a single serine protease inhibitor is present in the composition.

The diseases especially contemplated are, asthma, and skin diseases, such as eczema or psoriasis.

In one preferred embodiment, the pharmaceutiocal composition is in the form of an aerosol composition. This form is primarily intended for the treatment of asthma.

In another preferred embodiment, the pharmaceutical composition is in the form of a topical cream, primarily for the treatment of skin diseases.

In a still further preferred embodiment, the pharmaceutical composition includes an effective amount of a corticosteroid.

Serine protease inhibitors have been found to play a major role in the direct inactivation of the mediators of inflammation so that the normal wound healing process can be accelerated without interference from the excess of materials released at the site of inflammation. The almost immediate disappearance of pain and itch indicates that there can be a control of the kinins as well. A cocktail of serine protease inhibitors would therefore be useful to deactivate those mediators of inflammation which may not yet be recognized but are found in association with a particular inflammatory disease.

It is now recognized that in certain injuries or diseases, neutrophils, mast cells, T-cells and their mediators induce an inflammatory state resulting in a localized imbalance of elevated serine protease with a concomitant deficiency of their naturally occurring inhibitors despite normal serine protease inhibitor serum levels. Mast cells are critical in recruiting the cells (eosinophils, basophils and neutrophils) involved in the late phase reaction (LPR). Mast cell and neutrophil mediators appear to have a central role in the LPR Monocytes through the release of cytokines, interleukin -1,6 and tumor necroses factor further amplify the LPR Platelet activating factor, a mediator from mast cells, neutrophils and platelets is a potent bronchoconstrictor. Histamines are also released by the degranulation of mast cells as well as leukotriene T4 (LTB4) which play an important role in asthma. IgE upon activation by an antagonist causes degranulation of mast cells. Alpha 1-antitrypsin inhibits the mediators of mast cells and neutrophils, and also regulates IgE biosynthesis. The T-cell lymphokine glycosylation enhancing factor (GEF) is a serine protease that has been shown to enhance IgE response. By also inhibiting GEF there is a two level inhibition in the inflammatory cycle. The serine protease inhibitors decrease mast cell mediator release by inhibiting local IgE biosynthesis and T-cell lymphokine production. Serine proteases not only activate kinins and complements but also mediate tissue necrosis. The serine proteases, elastase and cathepsin G, have been shown to stimulate the production of platelet activating factor and LTB4.

As presently found, serine protease inhibitors are useful in the treatment of bum patients which not only experience pain and itch but have a problem in controlling the laydown of organized collagen because of elastase and cathepsin G; serine protease inhibitors particularly alpha 1-antitrypsin, permit the rapid growth of normal skin without degranulation.

The administration of serine protease inhibitors appears to be a viable alternative to the administration of steroids to reduce inflammation and to treat inflammatory skin conditions not treatable with steroids or to reduce the steriod requirement However, the combination with a cortiscosteroid has been found to provide a synergistic effect.

It has now been found that controlling the amount of the destructive enzymes at the site of inflammation can prevent proliferation of the disease, prevent associated tissue damage and promote healing. It has also been found that the administration of serine protease inhibitors which inactivate destructive proteases alone provide a major control of the symptoms of the disease or burns. However, since the cause of disease may be a result of more than one factors, the use of more than one protease inhibitor provides a better chance of success for early remission of the symptoms and for a prophylactic control of the symptoms associated with the disease. Serine protease inhibitors, for example, alpha 2-macroglobulin and C-reactive protein (CRP), when administered to the site of inflammation provides a reduction in swelling, pain and stiffness.

For chronic cases of dermatitis, a cocktail of serine protease inhibitors is preferably administered at the site of inflammation. The treatment can be followed with the addition of an appropriate steroid or antibiotic. There is a synergistic effect when the serine protease inhibitor is used in combination with a corticosteroid.

Among the corticosteroids which may be used in the present invention are triamcinolone acetonide, flurandrenolide, prednisone, amcinonide, dexamethasone, betamethasone valerate, halocinonide, clocortolone, hydrocortisone valerate, and the like.

Serine protease inhibitors have been found to play a major role in the direct inactivation of the mediators of inflammation so that the normal wound healing process can be accelerated without interference from the excess of materials released at the site of inflammation. The almost immediate disappearance of pain and itch indicates that there can be a control of the kinins as weD. Serine protease inhibitors, their analogs, salts or derivatives, appears to provide the quickest healing of psoriatic lesions when used in combination with a corticosteroid.

As presently found, serine protease inhibitors are useful in the treatment of chronic psoriasis patients which not only experience pain and itch but have a problem in controlling the laydown of organized collagen because of elastase and cathepsin G; serine protease inhibitors permit healing and the growth of normal skin. The presence of the steroids enhance the healing and promote a more rapid skin growth which is initiated by the serine protease inhibitors.

The serine protease inhibitors which are contemplated in the present invention are any of the inhibitors, their analogs, derivatives or salts of the human type which can inhibit mast cells or bind with any one or more of the protease derived from eosinophils, basophils and/or neutrophils such as elastase, cathepsin-G, tryptase, chymase, kinins, kallikrein, tumor necrosis factor, chymotrypsin, collagenase, inhibit IgE production and the like.

The serine protease inhibitors included in the present invention are human alpha 1-antitrypsin, alpha 2-macroglobulin, C-reactive protein, serum amyloid A protein, secretory leucocyte protease inhibitor,. The inhibitors of the invention may be natural or prepared by recombinant means. The recombinant may be glycosylated.

The use of alpha 1-antitrypsin has been especially useful in the treatment of the various inflammatory skin conditions including those which are induced by autoimmune disease, virus and bacterial infections. The serine protease inhibitors have also been found to cause vasoconstriction, which in inflammation, decreases swelling and redness and to eliminate pain and itching. This feature is especially useful in atopic dermatitis.

Alpha 1-antitrypsin has also been found especially useful in the treatment of bronchial and topical inflammatory conditions because of its association with elastase.

The drugs of the invention may be.derived from human blood or prepared by cloning, by conventional techniques utilizing an oligonucleotide probe or antibody probe, and the like. The recombinant gene product of the invention is especially useful since it is free of contaminating viruses when produced.

The analogs, salts and derivatives may be formed utilizing conventional techniques associated with other proteins without effecting the utility of the compound. There may be prepared the alkali metal salts, acid-addition salts, and esters similar to other proteins or peptides.

Some inflammation conditions are not immediately identifiable as to source and the factors which are involved to produce the different symptoms are not readily apparent. Therefore, it is desirable to administer in some case a combination or cocktail of serine protease inhibitors to provide a broad spectrum of drugs which can provide rapid relief of the different symptoms of inflammation. The most effective combination is alpha 1-antitrypsin and alpha 2-macroglobulin. Preferably, the combination is administered in a ratio of 1:1: to 2:1: either in a single unit or in separate dosage form.

When topically applied, a serine protease inhibitor such as alpha 1-antitrypsin in suitable composition form is useful in the treatment of inflammatory skin diseases such as psoriasis, eczema, acne, and the like.

The use of a non-aqueous lipid miscible carrier, for example, such as prepared with liposomes are particularly advantageous since they provided improved activity at the treatment sites.

The compositions of the invention are preferably administered to patients shoving an increase in IgE through a patch or serum test That is, the patient shows a positive allergic condition. These allergic patients having asthma respond quickly to therapy with alpha 1-antitrypsin when administered by inhalation form.

Preferably, the serine protease inhibitor is administered in an aqueous solution comprising 0.1 to 4.5% by weight of the inhibitor. A greater amount can be used but is generally not required.

The serine protease inhibitor binds with a stimulator of IgE synthesis or an inflammatory mediator of mast cell degranulation. These inhibitors further prevent protease from activating complement and kinins which cause the discomfiture associated with the disease.

The following examples further illustrate the practice of this invention, but are not intended to be limiting thereof. It will be appreciated that the selection of actual amounts of specific serine protease inhibitors to be administered to any individual patient (human or animal) will fall within the discretion of the attending physician and will be prescribed in a manner commensurate with the appropriate dosages will depend on the stage of the disease and like factors uniquely within the purview of the attending physician.

### EXAMPLE 1

A topical cream was prepared as follows:
A The following mixture was prepared:

| | |
|---|---|
| α₁ -antitrypsin | 1.0 g |
| Olive oil | 5.0 g |
| Cetanol | 2.0 g |
| Stearic acid | 5.0 g |
| Glycerin aliphatic acid ester | 12.0 g |
| Tween 60 | 0.5 g |

B. The following mixture was also prepared:

| | |
|---|---|
| Propylene glycol | 0.5 g |
| Methyl paraben | 0.1 g |
| Propyl paraben | 0.02 g |
| Purified water | to 100 g |

in total

The mixture of parts A and B were blended together by conventional means to give a total of 100 g. of 100% by weight topical cream which could be utilized for treatment of acne, eczema, psoriasis, or other inflammatory dermatological conditions. If desired secretory leucocyte protease inhibitor and/or alpha 2-macroglobulin as well as a corticosteroid may be added in an amount of 1.0 g to part A.

### EXAMPLE II

An olaginous anhydrous ointment was prepared with the following composition:

| | |
|---|---|
| Composition | % |
| α₁ -antitrypsin | 1.0 |
| Soy phosphatide | 4.0 |
| Plastibase 50W | 94.975 |
| Butylated hydroxytoluene | 0.025 |
| | 100.00 |

Other non-aqueous lipid miscible carriers may also be utilized. The composition may be used in combination with a topical corticosteroid.

### Example III (reference example)

In the treatment of colitis a 20% solution with alpha 1-antitrypsin may be prepared and administered as an enema. A similar result will be found with an secretory leucocyte protease inhibitor.

### Example IV

Microcrystalline alpha-1-antitrypsin is suspended in oleic acid and added into a metering aerosol cannister together with trichloromonofluoromethane and dichlorodifluoromethane so that the unit has a molecular proportion of alpha-1-antitrypsin to the propellant between 3:1 and 32. The unit delivers a quantity of drug equivalent to 42 mcg. The composition can be used in the treatment of asthma.

### Example V

Microcrystalline alpha-1-antitrypsin and alpha-1-antitrypsin is suspended in oleic acid and added into a metering aerosol cannister together with trichloromonofluoramethane and dichlorodifluoromethane so that the unit has a molecular proportion of drug to the propellant between 3:1 and 3:2.

### Example VI (reference example)

A composition for use in treating allergic rhinitis was prepared from the following ingredients.

| Ingredient | % wt |
|---|---|
| α₁-antitrypsin | 0.1 |
| 10% saline solution | 99.8 |
| antioxidant | 0.1 |

### Example VII

A pilot study was performed which consisted of a non-blinded trial using α₁-PI at a concentration of 20mg/ml in an aqueous solution in an alternate day schedule in conjunction with a 1% cream of α₁-PI (Stage I) and a 5% cream of α₁₋PI for maintenance therapy (Stage II). Prior to enrollment in this trial all 6 patients failed to respond to high potency top-ical steroids. Safety was gauged by careful clinical monitoring of subjective complaints, objective findings of erythema, edema and serial measurements of blood chemistries and complete blood counts. Wound healing was documented by serial photography. Written informed consent was obtained from each patient.

All six patients showed significant clinical improvement within 6 to 21 days of initiation of alternate-day therapy. α₁-PI stopped pain, pruritis and promoted tissue healing without scarring in all six patients. No adverse side effects of therapy were documented by clinical history, physical exam or by blood studies after 120 days of therapy. The results is seen in Table 1.

| Pt. # | Age /sex | Clinical Manifestions | Duration of Illness/ Previous Therapy | Lgth of Aqeous a1-PI Therapy | Therapy Response Time | | | (Stage II) Maint/ enance Therapy | Relapse Rate |
|---|---|---|---|---|---|---|---|---|---|
| 1 | 54/F | Digits and palms had erythematous, edematous, pruritic ulcerated and fisured lesions. Open wounds were both weeping Bleeding. Antecubital and popliteal fossae were eczematoid and lichenified. Decreased range of motion of hands. | 4 years Oral Prednisone IM Kenalog High Potency Top. Steroids Antibiotics Antiprurities Moisturizer | 45 days | ↓pain & Pruritis 30 Minutes trange of motion 24 hours reepith=Day 3 ulcer heal= Day 14 | | | 5% cream for 60 days | 0 |
| | | | | | | | | 5% cream & | |
| | | | | | | | | steroid (topical) 47 days | 0 |
| | | | | | | | | No therapy 40 days | ● |
| 2 | 36/F | Digits and palms were blistering, pruritic, oozing and bleeding. Decreased range of motion in both hands. Left hand had concomitant Lymphangitis with flares of her dermatitis. Mild blistering lesions of feet. | 5 years Oral Prednisone IM Kenalog High Potency Top. Steroids Antibiotics Coal Tar Preps Antipuruitics Moisturizers | 60 days | ↓pain % pruritis 30 Minutes trange of motion 24 hours Denude/Exfol= Day 3 Ulcer heal = Day 30 | | | 5% cream 21 days | 0 |
| | | | | | | | | 5% cream ● steroid 35 days | 0 |
| | | | | | | | | No therapy 50 days | 0 |
| 3 | 36/M | Dorsum of hand had blistering, Weeping, erythematous, edematous and pruritic Lesions. Occasional involvement of chest and arms. lesions would also go through cycles of crusting. | 3 years Oral Prednisone IM Kenalog Antipruritics High Potency Top. Steroids | 14 days | ↓pain & pruritis 30 minutes ↓eryth= Day 2 Appear Norm= Day 12 | | | No Therapy 90 days | 0 |
| 4 | 34/M | Single Chronic Erythematous, Blistering, scalding and pruritic lesion on right forearms. | 5 years Oral Prednisone Antipruritics High Potency, Topical Steroids | 42 days | ↓pain & pruritis 3 days ↓erythema day 4 Normal appearing skin day 6 | | | 5% cream 30 days | 0 |
| | | | | | | | | | |
| | | | | | | | | No Topical Steroids | ● |
| | | | | | | | | | |
| | | | | | | | | No Therapy 20 days | |
| 5 | 32/M | left hand involvement with fissuring, pruritis, scaling, minimal erythema and edema and decreased range of motion. | 10 years Oral Prednisone Moisturizers High Potency Topical Steroids | 30 days | ↓pain & pruritis 30 Minutes trange of motion 24 hours Healed skin 30 days | | | 5% cream | 0 |
| | | | | | | | | | |
| | | | | | | | | No Topical Steroids | |
| | | | | | | | | | |
| | | | | | | | | No Therapy 20 days | ● |
| 6 | 16/M | Bilateral hand involvement with extensive disease to distal phalanges: fissuring, bleeding, painful and pruritic Lesions. Decreased range of motion of hands. | 8 years Oral Prednisone Moisturizers Coal Tar Preps | 35 days | ↓pain & pruritis 4 days ↓erythema 7 days fissures healed day 7 | | | 5% cream % Topical Steroids 40 days | 0 |
| | | | | | | | | | |
| | | | | | | | | | |
| | | | | | | | | | |
| | | | | | | | | | |
| | | | | | | | | | |
| | | | | | | | | | |
| | | | | | | | | | |
| | | | | | | | | | |
| | | ● -only lab date that falls outside of normal limits is tabulated | | | | | | | |
| | | | | | | | | | |
| | | | | | | | | | |
| | | | | | | | | | |
| | | | | | | | | | |
| | | | | | | | | | |
| | | | | | | | | | |
| | | | | | | | | | |
| | | | | | | | | | |

## Claims

1. The use of alpha-1-antitrypsin, secretory leucocyte protease inhibitor, C-reactive protein, serum amyloid A protein and/or alpha-2-macroglobulin, their analogs, salts or derivatives which inhibit the degranulation of mast cells and/or have an affinity to the mediators of mast cells, for the preparation of a pharmaceutical, composition for administration to the site of disease or injury, of diseases implicated by mast cells, and their mediators, wherein the disease is asthma or a skin disease, and, if the disease is a skin disease, then only a single serine protease inhibitor is present in the composition.

2. The use according to claim 1, wherein said disease is asthma.

3. The use according to claim 1 or 2, wherein said pharmaceutical composition is an aerosol composition.

4. The use according to claim 1, wherein said disease is a skin disease.

5. The use according to claim 4, wherein said skin disease is eczema or psoriasis.

6. The use according to any one of claims 1, 4 or 5, wherein the pharmaceutical composition is a topical cream.

7. The use according to any one of claims 1-6, wherein an effective amount or a corticosteroid is, included in the pharmaceutical composition.

## Patentansprüche

1. Verwendung von alpha-1-Antitrypsin, sekretorische-Leukocyten-Proteaseinhibitor, C-reaktivem Protein, Serum-Amyloid-A-Protein und/oder alpha-2-Makroglobulin, deren Analoga, Salzen oder Derivaten, die die Degranulation von Mastzellen hemmen und/oder Affinität zu den Mediatoren von Mastzellen aufweisen, zur Herstellung einer pharmazeutischen Zusammensetzung zur Verabreichung am Ort einer Erkrankung oder Läsion von Erkrankungen, an denen Mastzellen und deren Mediatoren beteiligt sind, wobei die Erkrankung Asthma oder eine Hauterkrankung ist und wobei, wenn die Erkrankung eine Hauterkrankung ist, dann nur ein einziger Serinproteaseinhibitor in der Zusammensetzung vorhanden ist.

2. Verwendung nach Anspruch 1, wobei die Erkrankung Asthma ist.

3. Verwendung nach Anspruch 1 oder 2, wobei die pharmazeutische Zusammensetzung eine Aerosolzusammensetzung ist.

4. Verwendung nach Anspruch 1, wobei die Erkrankung eine Hauterkrankung ist.

5. Verwendung nach Anspruch 4, wobei die Hauterkrankung ein Ekzem oder Psoriasis ist.

6. Verwendung nach einem der Ansprüche 1, 4 oder 5, wobei die pharmazeutische Zusammensetzung eine topische Creme ist.

7. Verwendung nach einem der Ansprüche 1-6, wobei eine wirksame Menge eines Corticosteroids in der pharmazeutischen Zusammensetzung enthalten ist.

## Revendications

1. Utilisation d'α-1-antitrypsine, d'un inhibiteur de leucocyte-protéase sécrétoire, d'une protéine C-réactive, d'une protéine amyloïde A sérique et/ou d'α-2-macroglobuline, de leurs analogues, sels ou dérivés, qui inhibent la dégranulation des mastocytes et/ou ont une affinité pour les médiateurs des mastocytes, pour la préparation d'une composition pharmaceutique pour administration au site de la maladie ou de la blessure, pour les maladies impliquant les mastocytes et leurs médiateurs, dans laquelle la maladie est de l'asthme ou une maladie de peau et, si la maladie est une maladie de peau, alors uniquement un seul inhibiteur de sérine protéase est présent dans la composition.

2. Utilisation selon la revendication 1, dans laquelle ladite maladie est l'asthme.

3. Utilisation selon la revendication 1 ou 2, dans laquelle ladite composition pharmaceutique est une composition en aérosol.

4. Utilisation selon la revendication 1, dans laquelle ladite maladie est une maladie de peau.

5. Utilisation selon la revendication 4, dans laquelle ladite maladie de peau est l'eczéma ou le psoriasis.

6. Utilisation selon l'une quelconque des revendications 1, 4 ou 5, dans laquelle la composition pharmaceutique est une crème à usage local.

7. Utilisation selon l'une quelconque des revendications 1-6 dans laquelle une quantité efficace d'un corticostéroïde est incorporée dans la composition pharmaceutique.
